Europäisches Patentamt

European Patent Office

Office européen des brevets

(11) Veröffentlichungsnummer: **0 049 742**
**B1**

(12) **EUROPÄISCHE PATENTSCHRIFT**

(45) Veröffentlichungstag der Patentschrift:
06.11.85

(21) Anmeldenummer: 81105743.9

(22) Anmeldetag: 21.07.81

(51) Int. Cl.⁴: **C 12 P 19/18, C 12 N 11/00**

(54) Verfahren zur Herstellung von Isomaltulose (6-O-alpha-D-Gluco-pyranosido-D-fructose) mit Hilfe von immobilisierten Bakterienzellen.

(30) Priorität: 09.10.80 DE 3038219

(43) Veröffentlichungstag der Anmeldung:
21.04.82 Patentblatt 82/16

(45) Bekanntmachung des Hinweises auf die Patenterteilung:
06.11.85 Patentblatt 85/45

(84) Benannte Vertragsstaaten:
AT BE CH DE FR GB IT LI NL SE

(56) Entgegenhaltungen:
EP - A - 0 028 900
DE - B - 1 049 800
US - A - 3 779 869
US - E - 29 130

CHEMICAL ABSTRACTS, Band 79, Nr. 25, 24. Dezember 1973, Seite 205, Nr. 144877v, Columbus, Ohio, U.S.A.

(73) Patentinhaber: **Süddeutsche Zucker-Aktiengesellschaft, Maximilianstrasse 10, D-6800 Mannheim 1 (DE)**

(72) Erfinder: **Munir, Mohammad, Dr., Wormser Strasse 11, D-6719 Obrigheim 1 (DE)**

(74) Vertreter: **Körber, Wolfhart, Dr. et al, Patentanwälte Dipl.-Ing. H. Mitscherlich Dipl.-Ing. K. Gunschmann Dr.rer.nat. W. Körber Dipl.Ing. J. Schmidt-Evers Dipl.-Ing. W. Melzer Steinsdorfstrasse 10, D-8000 München 22 (DE)**

## Beschreibung

Gegenstand der Erfindung ist die kontinuierliche Umwandlung von Saccharose in Isomaltulose (Palatinose, 6-O-α-D-Glucopyranosido-D-fructose) mit Hilfe von immobilisierten Bakterienzellen.

Isomaltulose ist ein Zwischenprodukt zur Herstellung von Glucopyranosido-1,6-mannit (DE-AS 2 520 173) und Glucopyranosido-1,6-sorbit (Isomaltit, DE-PS 2 217 628). Beide Substanzen können als Zuckeraustauschstoffe eingesetzt werden.

Aus der deutschen Patentschrift 1 049 800 ist bekannt, dass Saccharose enzymatisch in Isomaltulose umgewandelt wird. Die Enzyme dazu sind mikrobiellen Ursprungs. Neben Protaminobacter rubrum sind weitere Bakterien wie Erwinia carotovora, Serratia marcescens, Serratia plymuthica und Leuconostoc mesenteroides zu dieser Umlagerung befähigt (S. Schmidt-Berg-Lorenz, W. Mauch, Zeitschrift für die Zuckerindustrie 14, 625–627 (1964); F.H. Stodola, 126th Meeting, amer. Chem. Soc., Sept. 1954, Abstracts of papers, S. 5 D; W. Mauch, S. Schmidt-Berg-Lorenz, Zeitschrift für die Zuckerindustrie 14, 309–315 und 375–383 (1964).

Weiterhin ist aus der DE-PS 2 217 628 bekannt, dass die enzymatische Umwandlung von Saccharose in Isomaltulose in einer 15- bis 40%igen Lösung unter starkem Rühren und aeroben Bedingungen bei 20 bis 37°C chargenweise oder kontinuierlich durchgeführt werden kann.

Ferner ist aus der DE-OS 2 806 216 bekannt, die Züchtung von isomaltulose-bildenden Mikroorganismen unter gleichzeitiger Umsetzung von Saccharose in Isomaltulose kontinuierlich durchzuführen.

Die vorgenannten bekannten Verfahren haben Nachteile wie z.B.

a) dass die lebenden und sich vermehrenden Zellkulturen einen Teil der zugesetzten Saccharose für die eigene Lebenstätigkeit (Wachstum und Vermehrung) verwenden bzw. veratmen, wodurch dieser Teil der Saccharose für die Umsetzung von Isomaltulose verloren geht;

b) dass die Umwandlung der Saccharose in Isomaltulose und die gleichzeitige Zellvermehrung eine Fermentation unter starker Rührung und Belüftung erfordern, was wiederum mit einem hohen Energieaufwand verbunden ist;

c) dass die Konzentration des Nährsubstrates mit 25% (DE-OS 2 806 216) bzw. 15 bis 40% (DE-PS 2 217 628) für die nachfolgende Gewinnung der Isomaltulose durch direkte Kristallisation zu gering ist; (Die umgesetzte Saccharoselösung muss nach Abtrennung der Zellmasse zunächst eingedampft werden, bevor sie einer Kristallisation unterworfen werden kann. So müssen aus 100 kg umgesetzter Saccharoselösung nach DE-OS 2 806 216 ca. 67 kg Wasser verdampft werden, ehe die Isomaltulose auskristallisiert.)

d) dass die Vermehrung der Zellmasse nicht nur eine geeignete Kohlenstoffquelle, sondern auch Stickstoff- und Nährsalze benötigt. (Bei dem oben erwähnten Verfahren erfolgt die Stickstoff- und Nährsalzzugabe dadurch, dass man anstelle von reinen Saccharoselösungen Zuckerlösungen geringerer Reinheit, wie sie in einer Zuckerfabrik anfallen, verwendet. Diese Verunreinigungen (Nichtzuckerstoffe) werden einerseits für das Zellwachstum unbedingt benötigt, andererseits verursachen sie bei der Kristallisation Isomaltuloseverluste durch Melassebildung.)

Die vorgenannten Nachteile sind prinzipiell vermeidbar, wenn es gelingt, die Umwandlung der Saccharose in Isomaltulose von der Zellvermehrung zu trennen und über längere Zeit zu stabilisieren.

Aus der älteren Anmeldung EP-A 0 028 900 ist ein Verfahren zur Herstellung von Isomaltulose durch enzymatische Umwandlung aus Saccharose mit Hilfe von toten immobilisierten Zellen von isomaltulose-bildenden Mikroorganismen in einem die Zellen enthaltenden Reaktor und anschliessende Kristallisation bekannt.

Demgegenüber besteht das erfindungsgemässe Verfahren darin, dass reine Saccharoselösungen mit Konzentrationen von 45 Gew.% bis 75 Gew.%, vorzugsweise 65 Gew.% bis 75 Gew.%, bei einer Temperatur von 40°C bis 65°C kontinuierlich durch den mit den Zellen gefüllten Reaktor geleitet werden.

Verfahren, die sich für die Immobilisierung der zur Umsetzung geeigneten Bakterienzellen als besonders vorteilhaft erwiesen haben, sind in den Unteransprüchen gekennzeichnet.

Die Vorteile des erfindungsgemässen Verfahrens sind:

a) Die isomaltulosehaltige Lösung kann ohne Voreindampfung sofort zur Kristallisation gebracht werden, wodurch eine erhebliche Energieeinsparung erzielt wird.

b) Die Verwendung toter Zellen erhöht die Ausbeute an Isomaltulose, da Saccharose nicht mehr für den Stoffwechsel der Bakterienmasse benötigt wird.

c) Die Isomaltulose-Ausbeute wird nochmals dadurch erhöht, dass man reine Saccharoselösungen einsetzen kann und somit eine Melassebildung weitgehend vermeidet.

d) Da Saccharoselösungen mit Konzentrationen von über 65 Gew.% mikrobiologisch stabil sind, kann auf eine Sterilisation des Substrats verzichtet werden.

e) Ein weiterer, nicht zu vernachlässigender Vorteil besteht darin, dass man aufgrund der höheren Substratkonzentration kleinere Volumina zu behandeln hat und somit mit kleineren Apparaturen auskommt.

f) Die Vermehrung der Bakterienmasse zur Herstellung von immobilisierten Zellen kann unter optimierten Wachstumsbedingungen erfolgen, und es brauchte demnach nicht mehr ein Kompromiss zwischen den optimalen Bedingungen für die Umsetzung zu Isomaltulose und für das Wachstum der Bakterien eingegangen zu werden.

g) Es kann ein billiges Nährsubstrat, z.B. verdünnte Melasselösung, zur Herstellung der Bakterienmasse eingesetzt werden.

Zur Gewinnung der anschliessend für das erfindungsgemässe Verfahren zu immobilisierenden Zellen erfolgt eine optimale Zellvermehrung in einem Nährmedium, das nur etwa 5 Gew.% Trockensubstanzgehalt aufweist. Das Nährmedium enthält Dicksaft (ein Zwischenprodukt der Zuckerindustrie), Maisquellwasser und $(NH_4)_2HPO_4$. Vorteilhaft ist jedoch der Einsatz eines wesentlich billigeren Nährsubstrates, das nur aus Zuckerrübenmelasse und $(NH_4)_2HPO_4$ besteht. Für die Herstellung dieses Nährmediums wird die Melasse mit dest. Wasser auf einen Trockensubstanzgehalt von 5 Gew.% verdünnt. Als zusätzliche Stickstoff- und Phosphatquelle gibt man zu 100 kg dieser Lösung 0,1 kg $(NH_4)_2HPO_4$ hinzu. Der pH-Wert wird mit Natron-, Kalilauge oder Salzsäure auf 7,2 eingestellt.

Eine Abimpfung eines isomaltulose-bildenden Mikroorganismus, z.B. Protaminobacter rubrum (CBS 574.77) wird mit 10 ml sterilem Nährsubstrat obiger Zusammensetzung abgeschwemmt in 200 ml desselben Nährmediums bei 29°C bebrütet. Sobald die Keimzahl in der Schüttelkultur $5 \cdot 10^9$ Keime/ml erreicht hat, wird die Schüttelkultur in einen Kleinfermenter mit Nährmedium obiger Zusammensetzung transferiert und mit möglichst hoher Belüftungsrate und Rührgeschwindigkeit bei 29°C weitervermehrt. Die Kontrolle der Vermehrung erfolgt wie bei der Schüttelkultur durch die Keimzahlbestimmung. Sobald die Keimzahl $5 \cdot 10^9$ Keime/ml erreicht hat, kann der Fermenter abgeerntet werden.

Für die Immobilisierung der erzeugten Zellen eignen sich grundsätzlich alle von I. Chibata (Immobilized Enzymes, John Wiley and Sons, New York, London, 1978) genannten Methoden zur Immobilisierung von ganzen Zellen. Folgende Methoden wurden als besonders geeignet befunden:

a) Der Fermenterinhalt wird entweder im Fermenter selbst oder in einem anderen Gefäss mit einer 1%igen Lösung eines kationenaktiven Flokkungsmittels versetzt. Die erforderliche Menge der Flockungsmittel-Lösung muss in einem Vorversuch ermittelt werden. Die ausgeflockte Zellmasse lässt man sedimentieren, schüttet die überstehende Flüssigkeit ab, wäscht den Niederschlag zweimal mit Phosphatpuffer (0,1 M, pH 7,0). Danach wird die Zellmasse zu einer extrudierbaren Paste entwässert, in Stränge extrudiert, getrocknet, gemahlen und abgesiebt.

b) Zu 10 l Fermenterbrühe gibt man unter langsamem Rühren 0,5 bis 2,0 l einer 0,2%igen Chitosanlösung hinzu. Die ausgeflockte Zellmasse wird mit Phosphatpuffer (0,1 M, pH 7,0) gewaschen, in einer Zentrifuge, z.B. Becher- oder Vollmantelzentrifuge, entwässert, in Stränge extrudiert, getrocknet, gemahlen und abgesiebt.

c) Die Zellmasse wird in einer Zentrifuge von der Fermenterbrühe abgetrennt. 5 bis 20 g dieser Zellsuspension (Trockensubstanzgehalt ca. 10%) werden in 45 bis 60 ml Na-alginatlösung (8 g in 100 ml) suspendiert und durch eine Kanüle (Durchmesser 0,55 mm) in 2% $CaCl_2$-Lösung hineintropfen gelassen. Die entstandenen Perlen werden ca. 15 Minuten in $CaCl_2$-Lösung weitergerührt und dann mit Wasser ausgewaschen. Die Perlen werden ca. 20 h bei Raumtemperatur getrocknet.

d) Die Zellmasse wird in einer Zentrifuge von der Fermenterbrühe abgetrennt, mit Phosphatpuffer (0,1 M, pH 7,0) gewaschen und lyophilisiert.

15 g Cellulosediacetat werden in 100 ml eines Gemisches aus Dimethylsulfoxid und Aceton (6:4) bei 90°C unter Rückfluss gelöst. Nach dem Abkühlen auf 30°C gibt man 3 bis 30 g lyophilisierte Zellen hinzu. Diese Suspension wird mit Hilfe einer Injektionsspritze (Kanüle 0,8 mm) in Wasser bei Raumtemperatur hineintropfen gelassen. Die gebildeten Perlen werden getrocknet.

e) Die Zellmasse wird in einer Zentrifuge von der Fermenterbrühe abgetrennt. 5 g dieser Zellmasse werden in 5 ml physiologischer Kochsalzlösung bei 45 bis 50°C suspendiert. 1,7 g K-Carrageenan (löslicher Polysaccharidschwefelester) werden in 34 ml physiologischer Kochsalzlösung bei 45 bis 60°C gelöst. Beide Lösungen werden gemischt und 30 Minuten bei 10°C gehalten. Das entstandene Gel wird in kalter 0,3 molarer KCl-Lösung gehärtet und dann in ungefähr 3 mm grosse Stücke gebrochen.

f) Die Fermenterbrühe wird entweder im Fermenter selbst oder in einem anderen Gefäss zuerst mit einer 1%igen Lösung eines kationenaktiven Flockungsmittels und dann mit einer 1%igen Lösung eines anionenaktiven Flockungsmittels versetzt. Die erforderliche Menge der Flockungsmittel muss in einem Vorversuch ermittelt werden. Die ausgeflockte Zellmasse wird absetzen gelassen mit Phosphatpuffer (0,1 M, pH 7,0) gewaschen, zu Strängen extrudiert, getrocknet, gemahlen und abgesiebt.

Die nach den vorstehend angegebenen Methoden hergestellten Präparate müssen, um ein Ausbluten der Zellen zu verhindern, vernetzt werden. Für die Vernetzung verwendet man bifunktionelle Reagenzien, wie z.B. Glutaraldehyd. Die üblicherweise verwendete Glutaraldehydkonzentration von 2,5 bis 5,0% bei einer Kontaktzeit von 30 bis 45 Minuten kann für isomaltulose-bildende Mikroorganismen nicht eingesetzt werden. Es wurde gefunden, dass alle immobilisierten Präparate unter diesen Bedingungen völlig inaktiviert werden. Als optimale Vernetzungsbedingungen wurden für das vorliegende Verfahren eine Konzentration von 0,1% Glutaraldehyd und eine Behandlungsdauer von 10 Minuten festgestellt.

Die nach einem der vorstehend beschriebenen Methoden immobilisierten und vernetzten Zellen können in einen geeigneten Säulenreaktor gefüllt werden. Dieser Reaktor muss temperierbar sein und einen Durchmesser zum Betthöhen-Verhältnis von 1:1 bis 1:20 haben, vorzugsweise 1:1 bis 1:10, insbesondere 1:1,5 bis 1:5. Reine Saccharoselösung (65 bis 75 Gew.%) wird bei einer Temperatur von 40 bis 60°C entweder von oben nach unten oder von unten nach oben durch die Säule gepumpt. Dabei wird die Strömungsgeschwindigkeit so eingestellt, dass eine Kontaktzeit von 1 bis 10 Stunden, vorzugsweise 4 bis 7 erreicht wird (das entspricht 0,2–0,8 g Saccharose, vorzugsweise 0,4–0,6 g Saccharose pro g immobilisierte Zelle (trocken) pro Stunde). Die eingesetzte Saccharose wird dabei voll umgesetzt, wobei ca. 1% Glucose, 4% Fructose, 82% Isomaltulose und 13% 1-O-α-D-Glucopyranosido-D-fructose entstehen.

Ein Teil der Isomaltulose kann durch einfaches Abkühlen der Lösung kristallin gewonnen werden. Durch Eindampfen und Kühlen kann man aus der Mutterlauge eine weitere Menge Isomaltulose herauskristallisieren, so dass insgesamt etwa 91,5% der entstandenen Isomaltulose kristallin gewonnen werden können.

Eine vorteilhafte Ausgestaltung des Verfahrens besteht darin, dass man die Saccharoselösung schneller durch die Säule leitet, so dass nur etwa 75 bis 80% der Saccharose umgesetzt werden. Aus dem Produktstrom kann man einen Teil der Isomaltulose durch Kühlen auskristallisieren und den Rest nach Ergänzung mit frischer Saccharoselösung nochmals über die Säule leiten. Der Vorteil dieser Arbeitsweise liegt darin, dass durch die kürzere Kontaktzeit die Bildung von 1-O-α-D-Glucopyranosido-D-fructose unterdrückt wird, und man im Endeffekt eine grössere Ausbeute des gewünschten Produktes Isomaltulose erzielen kann.

Die Arbeitsweise des Verfahrens wird anhand von zwei Beispielen näher erläutert:

Beispiel 1
a) Von einer Abimpfung des Stammes Protaminobacter rubrum (CBS 574.77) werden Zellen mit 10 ml eines sterilen Nährsubstrates, bestehend aus 8 kg Dicksaft aus einer Zuckerfabrik (Trockensubstanzgehalt = 65%), 2 kg Maisquellwasser, 0,1 kg $(NH_4)_2HPO_4$ und 89,9 kg dest. Wasser (bei Bedarf auf pH 7,2 eingestellt) abgeschwemmt. Diese Suspension dient als Impfgut für die Schüttelmaschinen-Vorkultur in 1-l-Kolben mit 200 ml Nährlösung obiger Zusammensetzung.

Nach einer 30stündigen Bebrütungszeit bei 29°C werden mit je 20 Kolben (4 l) 16 l Nährlösung obiger Zusammensetzung in einem 30-l-Kleinfermenter beimpft und bei 29°C mit 20 l Luft pro Minute und einer Rührgeschwindigkeit von 350 UpM fermentiert. Die anwachsende Keimzahl wird mikroskopisch bestimmt. Nach Erreichen von $5 \cdot 10^9$ Keimen/ml wird der Fermenterinhalt in ein anderes Gefäss transferiert, dort mit einem kationenaktiven Flockungsmittel (wie z.B. Primafloc C7 von Rohm & Haas, Philadelphia/USA) versetzt. Die ausgeflockten Zellen werden absetzen gelassen, abdekantiert, mit 0,1 M Phosphatpuffer pH 7,0 gewaschen und an der Zentrifuge entwässert. Die Masse wird dann zu Strängen extrudiert, an der Luft getrocknet und gemahlen.

b) die Siebfraktion 0,3 bis 0,8 mm aus dem oben gewonnenen Präparat wird in einer 0,1%igen Glutaraldehydlösung 10 Minuten gerührt, mit Phosphatpuffer (0,1 M, pH 7,0) gewaschen und unter Wasser in eine temperierbare Säule gefüllt. Die Säule wird dann auf 50°C erwärmt und mit einer 70 gew.%igen Saccharoselösung kontinuierlich durchströmt. Die Strömungsgeschwindigkeit wird dabei so eingestellt, dass am Ende der Säule keine Saccharose mehr nachweisbar ist. Die so gewonnene Isomaltuloselösung wird einem Kühlungskristallisator zugeführt, auf 20°C gekühlt und die auskristallisierte Isomaltulose an Siebkorbzentrifugen von der Mutterlauge abgetrennt.

Beispiel 2
Von einer Abimpfung des Stammes Protaminobacter rubrum (CBS 574.77) werden Zellen mit 10 ml eines sterilen Nährsubstrates bestehend aus 6,25 kg Rübenmelasse (Trockensubstanzgehalt = 80%). 0,1 kg $(NH_4)_2HPO_4$ und 93,65 kg dest. Wasser (falls erforderlich, mit HCl auf pH 7,2 eingestellt) abgeschwemmt. Diese Suspension dient als Impfgut für die Schüttelmaschinen-Vorkultur in 1-l-Kolben mit 200 ml Nährlösung obiger Zusammensetzung. Nach einer 30stündigen Bebrütungszeit bei 29°C werden mit je 20 Kolben (4 l) 16 l Nährlösung obiger Zusammensetzung in einem 30-l-Kleinfermenter beimpft und bei 29°C mit 20 l Luft pro Minute und einer Rührgeschwindigkeit von 350 UpM fermentiert.

Es wird weiter so verfahren, wie in Beispiel 1a und 1b beschrieben.

**Patentansprüche**

1. Verfahren zur Herstellung von Isomaltulose (6-O-α-D-Glucopyranosido-D-fructose) durch enzymatische Umwandlung aus Saccharose mit Hilfe von toten immobilisierten Zellen von isomaltulose-bildenden Mikroorganismen in einem die Zellen enthaltenden Reaktor und anschliessende Kristallisation, dadurch gekennzeichnet, dass reine Saccharoselösungen mit Konzentrationen von 45 Gew.% bis 75 Gew.%, vorzugsweise 65 Gew.% bis 75 Gew.%, bei einer Temperatur von 40°C bis 65°C kontinuierlich durch den mit den Zellen gefüllten Reaktor geleitet werden.

2. Verfahren nach Anspruch 1, dadurch gekennzeichnet, dass es sich bei den verwendeten Mikroorganismen um Protaminobacter rubrum (CBS 574.77), Serratia plymuthica (ATCC 15928), Serratia marcescens (NCIB 8285) und Leuconostoc mesenteroides [NRRL B-512F (ATCC 10830a)], vorzugsweise um Protaminobacter rubrum (CBS 574.77), handelt.

3. Verfahren nach Anspruch 1, dadurch gekennzeichnet, dass die immobilisierten Zellen mit einem bifunktionellen Reagens, wie Glutaraldehyd oder Cyanurhalogenid, vernetzt werden.

4. Verfahren nach Anspruch 1, dadurch gekennzeichnet, dass die Zellen durch Ausflockung mit einem kationenaktiven Flockungsmittel immobilisiert werden.

5. Verfahren nach Anspruch 4, dadurch gekennzeichnet, dass die Zellen durch Ausflockung mit Chitosan immobilisiert werden.

6. Verfahren nach Anspruch 1, dadurch gekennzeichnet, dass die Zellen durch Einschluss in einer Calciumalginat-Matrix immobilisiert werden.

7. Verfahren nach Anspruch 1, dadurch gekennzeichnet, dass die Zellen durch Einschluss in Cellulosediacetat bzw. Cellulosetriacetat immobilisiert werden.

8. Verfahren nach Anspruch 1, dadurch gekennzeichnet, dass die Zellen durch Einschluss in K-Carrageenan-Gel immobilisiert werden.

9. Verfahren nach Anspruch 1, dadurch gekennzeichnet, dass die Zellen durch Ausflockung mit kationenaktivem oder anionenaktivem Flokkungsmittel oder mit einer Kombination aus beiden immobilisiert werden.

## Claims

1. Process for preparing isomaltulose (6-O-$\alpha$-D-glucopyranosyl-D-fructose) by enzymatic conversion of saccharose using dead, immobilised cells of an isomaltulose-forming microorganism in a reactor containing the cells, followed by crystallisation, characterised in that a pure saccharose solution having a concentration of from 45 to 75% by weight, preferably 65 to 75% by weight, is passed continuously at a temperature of 40 to 65°C through the reactor filled with the cells.

2. Process according to claim 1, characterised in that the microorganism used is Protaminobacter rubrum (CBS 574.77), Serratia plymuthica (ATCC 15928), Serratia marcescens (NCIB 8285) or Leuconostoc mesenteroides [NRRL B-512F (ATCC 10830a)], preferably Protaminobacter rubrum (CBS 574.77).

3. Process according to claim 1, characterised in that the immobilised cells are cross-linked with a bifunctional reagent such as glutaraldehyde or a cyanuric halide.

4. Process according to claim 1, characterised in that the cells are immobilised by flocculation with a cation-active flocculating agent.

5. Process according to claim 4, characterised in that the cells are immobilised by flocculation with chitosan.

6. Process according to claim 1, characterised in that the cells are immobilised by occlusion in a calcium alginate matrix.

7. Process according to claim 1, characterised in that the cells are immobilised by occlusion in cellulose diacetate or cellulose triacetate.

8. Process according to claim 1, characterised in that the cells are immobilised by occlusion in K-carrageenan gel..

9. Process according to claim 1, characterised in that the cells are immobilised by flocculation with a cation-active or anion-active flocculating agent or with a combination of the two.

## Revendications

1. Procédé de fabrication d'isomaltulose (6-O-$\alpha$-D-glycopyranosido-D-fructose) par transformation enzymatique à partir du saccharose, à l'aide de cellules mortes immobilisées de micro-organismes générateurs d'isomaltulose dans un réacteur contenant les cellules, suivie d'une cristallisation, caractérisé en ce que les solutions pures de saccharose ayant des concentrations de 45% à 75% en poids et, de préférence, de 65% à 75% en poids, passent d'une manière continue, à une température de 40 à 65°C, à travers le réacteur rempli avec les cellules.

2. Procédé suivant la revendication 1, caractérisé en ce que les micro-organismes utilisés sont le protaminobacter rubrum (CBS 574.77), le serratia plymuthica (ATCC 15928), le serratia marcescens (NCIB 8285) et le leuconostoc mesenteroides [NRRL B 512 F (ATCC 10830a)], mais de préférence le protaminobacter rubrum (CBS 574.77).

3. Procédé suivant la revendication 1, caractérisé en ce que les cellules immobilisées sont réticulées avec un réactif bifonctionnel, comme le glutaraldéhyde ou le cyanurhalogénide.

4. Procédé suivant la revendication 1, caractérisé en ce que les cellules sont immobilisées par floculation avec un floculant actif cationiquement.

5. Procédé suivant la revendication 4, caractérisé en ce que les cellules sont immobilisées par floculation avec du chitosan.

6. Procédé suivant la revendication 1, caractérisé en ce que les cellules sont immobilisées par inclusion dans une matrice d'alginate de calcium.

7. Procédé suivant la revendication 1, caractérisé en ce que les cellules sont immobilisées par inclusion dans du diacétate de cellulose ou du triacétate de cellulose.

8. Procédé suivant la revendication 1, caractérisé en ce que les cellules sont immobilisées par inclusion dans du gel K-carrageenan.

9. Procédé suivant la revendication 1, caractérisé en ce que les cellules sont immobilisées par floculation avec un floculant cationiquement ou anioniquement actif, ou avec une combinaison des deux.